# EUROPEAN PATENT APPLICATION

(11) **EP 4 621 803 A1**
(43) Date of publication of application: **24.09.2025**
(21) Application number: 24305428.5
(22) Date of filing: 22.03.2024
(51) Int. Cl.: G16H 50/30

(54) **METHOD FOR IDENTIFYING PATIENTS IN NEED FOR ICI-INDUCED MYOTOXICITY TREATMENT**

(71) Applicant: Assistance Publique - Hôpitaux de Paris, 75012 Paris (FR); Sorbonne Université, 75006 Paris (FR); Institut National de la Santé et de la Recherche Médicale (INSERM), 75013 Paris (FR)
(72) Inventor: SALEM, Joe-Elie, 75015 PARIS (FR)
(74) Representative: Flesselles, Bruno F.G.

(57) **Abstract**

The invention pertains to the field of cancer treatment by Immune Checkpoint Inhibitors (ICls) and to the risk stratification and personalized treatment of patients having ICI-induced myotoxicity.

## Description

The invention pertains to the field of cancer treatment by Immune Checkpoint Inhibitors (ICI) and to the identification, stratification and personalized treatment of patients having ICI-induced myotoxicity notably polymyositis potentially affecting the heart (myocarditis) and/or peripheral muscles (myositis) potentially mimicking myasthenia-gravis.

Immune checkpoint inhibitors (ICI) work by blocking proteins on immune cells (checkpoints) that prevent them from attacking cancer cells. They have emerged as a mainstay of cancer therapy with widespread adoption across multiple tumor types and clinical settings. Almost 50% of patients with cancer are currently eligible for ICI therapy (1). ICI activate the immune system for anti-tumor efficacy but cause immune related adverse events (irAE) in 70-90% of patients (2-4). IrAE can affect any organ including cardio-muscular tissues and range from mild to fatal (1,5-8) ICI-myocarditis, which occurs in 0.3-1% of ICI recipients, is the most fatal irAE with an estimated mortality of 30-50% in seminal studies (5,7,9). However, ICI-myocarditis severity can be heterogeneous; while asymptomatic cases are increasingly recognized, others patients experience life-threatening arrhythmias, cardiogenic shock, or severe concomitant myositis with respiratory muscle failure (5,10). Triage and management of this rare condition remains a significant challenge even for advanced multidisciplinary teams.

Among approved immune checkpoint inhibitors (ICI), one can cite the ones that target the molecules CTLA4 (Cytotoxic T-Lymphocyte associated protein 4), PD-1 (the transmembrane programmed cell death 1 protein, also called PDCD1 and CD279), and PD-L1, which is the PD-1 ligand (or CD274). PD-1 acts as a key regulatory role on T-cell activities, and cancer-mediated upregulation of PD-L1 on the cancer cell surface may inhibit T-cells recognition and action against these cells. in particular, antibodies against PD-1 or PD-L1 block the interaction between these proteins and allow T-cells to attack the tumor. CTLA-4 (CD152), which is a protein receptor constitutively expressed in regulatory T cells and upregulated in conventional T-cells after activation, and acts as an inhibitory switch of the immune system when it binds to CD80 or CD86 expressed on the surface of antigen-presenting cells may also be targeted.

As other targets of ICI, one can also cite
- Lymphocyte activation gene 3 (LAG-3), found on the surface of T cell
- T cell immunoglobulin and mucin domain-containing protein 3 (TIM-3), found on the surface of T cells and other immune cells
- Indoleamine 2,3-dioxygenase 1 (IDO1), an enzyme involved in the metabolism of the amino acid tryptophan
- B7-H3 (CD276):
- V-domain Ig suppressor of T cell activation (VISTA)
- T cell immunoreceptor with Ig and ITIM domains (TIGIT)
- Siglec-15
- CD47
- BTLA (B and T lymphocyte attenuator)
- CD80
- Tumor necrosis factor receptor superfamily member 9 (TNFRSF9 or 4-1 BB).

The immune checkpoint inhibitor used for the patient's treatment before occurrence of the adverse effect is any such drug in this class.

In particular, one can cite:
PD-1 inhibitors : IgG4 PD1 antibody nivolumab, pembrolizumab, partalizumab (PDR001) developed by Novartis, pidilizumab developed by Cure Tech, AMP-224 or AMP-514 both developed by GlaxoSmithKline, cemiplimab developed by Regeneron and Sanofi, toripalimab developed by Shanghai Junshi, spartalizumab, developed by Novartis, cetrelimab (JNJ-63723283) developed by Janssen, dostarlimab or sasanlimab (PF-06801591) developed by Pfizer.
PD-L1 inhibitors: atezolizumab developed by Roche Genentech, avelumab developed by Merck Serono and Pfizer or durvalumab developed by AstraZeneca.
Anti-CTLA4: ipilimumab or tremelimumab.
LAG-3 inhibitors: Relatlimab (BMS-986016), IMP761 an antibody-based LAG-3 inhibitor, eLAG-3Fc, IPI-389601.

Toxicities (irAE) may arise from use of ICI. These generally are *de novo* toxicities, i.e. not related to a preexisting immune condition. Although they can affect any organ, the most life-threatening irAE are ICI-induced myotoxicities, including myocarditis or myositis potentially mimicking myasthenia gravis.

Because ICI-myocarditis is rare and difficult to diagnose, prognostic studies to date have relied on pharmacovigilance datasets or small cohorts of less than 150 patients (7,9,11,12). These analyses had limited adjustment for covariates and their results vary widely. Proposed predictors of severity include combination ICI treatment, elevated biomarkers, conduction delays, systolic left ventricular dysfunction, inadequate immunossupressant therapy, and simultaneous myasthenia or myositis irAE. Reconciling these findings is challenging as these features often coexist. Consequently, major knowledge gaps exist in selecting the appropriate level of care and immunosuppression strategy for ICI-myocarditis. To address these deficits, a web-based platform was leveraged to build a multicenter registry with approximately 750 adjudicated cases of ICI-myocarditis. The invention is linked to the identification of predictors of severe outcomes in ICI-myocarditis to guide treatments. Using these results, a risk score was generated to assist clinicians with risk stratification. the present specification thus discloses, for the first time, that it is possible to identify biological and clinical markers that can be combined and provide a score indicative of magnitude of risk of severe cardiomyotoxicity. Consequently, this also makes it possible to identify patients at very low risk of major cardio-myotoxic events (MACE), and subsequently death related to the myotoxicity. Instead of administering to all patients' high doses of immunossupressant (e.g corticosteroids boluses), as currently recommended (ESC Guidelines on cardio-oncology, European Society of Cardiology 2022), the methods herein disclosed are appropriate to identify MACE within 30 days of ICI-induced myotoxicity diagnosis. With this information, the physician can adjust and initiate immunosuppressive treatments or not, as appropriate, to lower the patient's risk.

As understood herein, major cardio-myotoxic events, is defined as any of the following: 1) severe heart failure requiring either intravenous medicines or mechanical circulatory support 2) severe arrhythmia defined as sustained ventricular arrythmia, complete heart block, sudden cardiac death, use of atropine or isoproterenol, and/or use of pacemaker/defibrillator 3) respiratory failure due to myositis-related respiratory muscles failure requiring ventilation (leading to hypercapnia) and/or 4) death due to these conditions (henceforth referred to as cardio-myotoxicities) .

In the context of the present application, an ICI-treated patient with ICI-induced myotoxicity is classified as having an increased risk of MACE if the risk of the patient is higher than the general risk of MACE for ICI-treated patients and with ICI-induced myotoxicity.

It is indeed to be noted that the methods herein disclosed are performed on ICI-treated patients who have been diagnosed with a myotoxicity. One understands that, if the general risk of MACE for patients with ICI-induced myotoxicity is x % (generally x% is about 30%), the risk of patients that are identified as having a higher risk by the methods herein disclosed would be higher than x %.

A risk of x % within a population indicates that, in the population, x % of the patients present the defined phenotype (here MACE, such event generally occurring within 30 days after presentation (medical consult) of the patient with diagnosis of the ICI-induced myotoxicity).

One can also note that, for some embodiments, the methods herein disclosed make it possible to classify the patients in two classes (at risk of MACE / not at risk of MACE). Indeed, as will be seen in the examples, it is possible to determine thresholds (cutoffs) so that the patients assigned to the "*not at risk of myotoxicity-related* MACE" have indeed no or a very low risk (less than 3% or even less than 1%) of MACE and moreover of myotoxicity-related death, and would not need treatment. The other patients would be treated and hence would be protected from the occurrence of MACE (the risk of a treated patient in the "at risk" group would become lower than the risk of untreated patient in the "at risk group").

One can also note that, in some embodiments, the method provides quantitative results, i.e. that the higher the end-value, the higher the risk is. Thus, the magnitude of risk is incremental with the score (end-value).

The invention thus relates to a method for determining whether a patient under ICI and presenting an induced myotoxicity has an increased risk or an absence of risk of MACE comprising
- assigning a value to clinical, biological and/or radiological markers obtained from the patient
- combining the values through a mathematical function,
- obtaining an end-value,
wherein the end-value is indicative of the increased risk or of the absence of risk of MACE. The end-value may also be compared to a reference, notably to ascertain the magnitude of risk.

This method is performed e*x vivo* and can be computer-implemented.

In some embodiments, the higher the end-value, the higher the risk.

In particular, the myotoxicity is a myocarditis (inflammation of the heart muscle (myocardium)) or a myositis potentially mimicking myasthenia gravis.

The diagnosis of immune checkpoint inhibitor-induced myocarditis is sometimes challenging and requires the multimodal acquisition of clinical, biological, and imaging features corroborating the diagnosis.

As indicated by Fenioux et al (Nat Med. 2023;29(12):3100-3110), ICI-induced myocarditis diagnosis may be performed by
a) excluding any other diagnosis/explanations for the myocarditis, and particularly cardiotoxicity of another liable drug used in combination with the ICI
b) diagnosing definite, probable, and possible myocarditis if the patient presents
   i. Definite myocarditis (any one of the below situations is sufficient)
      - Definite cardiac pathology
      - Definite CMR + syndrome + (biomarker or ECG or WMA)
      - WMA + syndrome + biomarker + ECG + negative angiography
      - Definite muscular pathology + (suggestive/definite CMR or WMA or ECG or suggestive cardiac pathology) + biomarker
      - Suggestive (muscular or cardiac) pathologies + (suggestive/definite CMR or WMA or ECG) + biomarker + syndrome
   ii. Probable myocarditis (any one of below situations is sufficient)
      - Definite CMR + (biomarker or ECG or WMA) and no syndrome
      - Suggestive CMR + biomarker + (syndrome or ECG or WMA)
      - WMA + syndrome + biomarker + ECG and no available angiography
      - Suggestive muscular pathology + (suggestive CMR or WMA or ECG) + (biomarker or syndrome)
      - Suggestive cardiac pathology + (biomarker or syndrome or ECG)
      - Syndrome + biomarker + ECG + negative angiography + no WMA + normal CMR + normal cardiac pathology
   iii. Possible myocarditis (any one of the below situations)
      - Suggestive CMR + biomarker with no (syndrome or ECG or WMA)
      - Suggestive/aspecific lesions on muscular pathology + biomarker
      - Suggestive cardiac pathology
      - Biomarker + (syndrome or ECG) and no alternative diagnosis*
      with the following detailed criteria required depending on modality used for diagnosis of ICI-myocarditis:
      - Cardiac pathology (endomyocardial or autopsy samples):
         ∘ Definite: Abnormal lympho-histiocytic inflammatory cells infiltrate (CD3+ cells ≥7/mm² or CD68+ cells ≥4/mm²) AND cardiomyocytes necrosis.
         ∘ Suggestive (borderline): Abnormal lympho-histiocytic inflammatory cells infiltrate (CD3+ cells ≥7/mm² or CD68+ cells ≥4/mm²) WITHOUT cardiomyocytes necrosis.
      - Cardiac magnetic resonance imaging (analysis based on T1 & T2 properties of cardiac tissue):
         ∘ Definite: Tissue imaging identifying unknown abnormalities in cardiac segments in both T1 (native T1 ± extracellular volume ± presence of late gadolinium enhancement) AND T2 derived maps and sequences.
         ∘ Suggestive (borderline): Tissue imaging identifying unknown abnormalities in cardiac segments in either T1 OR T2 derived maps and sequences.
      - Peripheral muscle pathology (skeletal muscles or autopsy samples):
         ∘ o Definite: Abnormal endomysial lympho-histiocytic inflammatory cells infiltrate AND abnormal class 1 human leukocyte antigen overexpression on myocytes AND myocytes necrosis.
         ∘ o Suggestive (borderline): Abnormal endomysial lympho-histiocytic inflammatory cells infiltrate AND abnormal class 1 human leukocyte antigen overexpression on myocytes WITHOUT myocytes necrosis.
         ∘ Non-specific lesions: Abnormal class 1 human leukocyte antigen overexpression on myocytes WITHOUT abnormal endomysial lympho-histiocytic inflammatory cells infiltrate WITHOUT myocytes necrosis.

*Abbreviations:* Biomarker: identification of unknown increase of troponin assay above its 99th percentile upper reference limit (as identified by the manufacturer); CMR: cardiac magnetic resonance imaging (analysis based on T1 & T2 properties of cardiac tissue); ECG: electrocardiogram (12-leads 10 seconds) identifying an unknown abnormality (atrio-ventricular or sinus blocks, bundle branch blocks, ST-T wave changes, micro-voltage, ventricular or supra-ventricular arrhythmias, pathological Q-waves); Syndrome: appearance on ICI of any of the following abnormalities (dyspnea, chest pain, diplopia, ptosis, myalgia, muscular weakness, fatigue, dysphonia, dysphagia, abdominal paradox, syncope, faintness, palpitations, ventricular hyperexcitability on electrocardiographic holter/telemetry (>5% of non-sinus ventricular rhythms), and respiratory muscle involvement probable or definite; WMA: identification of unknown wall motion abnormality or left ventricular ejection fraction<50% by cardiac imaging.
* Acute coronary syndrome and ICI-myocarditis may co-occur at presentation. ICI-myocarditis should be reassessed after revascularization particularly if this has not led to resolution of alteration in biomarkers, ECG or syndrome or if syndrome is including peripheral muscle involvement not explained by cardiac ischemia (diplopia, ptosis, myalgia, myalgia, muscle weakness, dysphagia, dysphonia).

The methods herein disclosed can be used for any patient with definite, probable, or possible myocarditis.

According to Solimando et al (Int J Mol Sci. 2020; 21(9): 3054), ICI-induced myositis (polymyositis/dermatomyositis) may be diagnosed by:
- Neurological and rheumatological anamnesis, rheumatological (inspection of the skin to identify signs suggestive of dermatomyositis) and neurological (muscle strength determination) objective examinations
- Blood chemistry tests including:
   i. CPK (creatine phosphokinase), transaminases (AST, aspartate aminotransferase; ALT, alanine aminotransferase), LDH (Lactate dehydrogenase), aldolase
   ii. Cardiac enzymes (to identify possible concomitant myocarditis)
   iii. Markers of inflammation (ESR, erythrocyte sedimentation rate; CRP, C-reactive protein)
   iv. Search for anti-AChR (acetylcholine receptor) auto-antibodies (to identify possible concomitant MG (myasthenia gravis) and for antibodies causing neurological syndromes and paraneoplastic myositis
- Potential neurophysiological examination (needle EMG (electromyoneurography), neuromuscular plaque determination to identify possible concomitant MG, or a nerve conduction study to identify possible concomitant neuropathy)
- Potential muscle MRI (magnetic resonance imaging) or tissue biopsy if the diagnosis is uncertain

Rheumatological or neurological evaluation can also help for the diagnosis and for determining the grade of the disease.

The methods herein disclosed are thus to be performed on such patients that
- Receive or have received a treatment by ICI
- Has been diagnosed with a myotoxicity (myocarditis or myositis, potentially mimicking myasthenia-gravis).

It is not necessary that the patients still receive the ICI at the time the methods are performed. Indeed, such treatment may have been withheld by the physicians upon occurrence of the myotoxicity. However, the patients have received such treatment, the most recent dose of ICI having been received in the 3 months preceding the diagnosis of ICI-myotoxicity.

Various markers can be used in the methods herein described.

On can cite presence of active thymoma in the patient, presence of cardio-muscular symptoms, voltage on presenting electrocardiogram (such as QRS, T-wave or P-wave), concentration of at least one circulating blood marker selected from ALT (Alanine aminotransferase), AST (Aspartate aminotransferase), LDH (lactate desydrogenase), aldolase, erythrocyte sedimentation rate, level of CRP, level of circulating cardiac and/or muscular enzymes (troponin and/or creatinine kinase), presence of anti-AChR (acetylcholine receptor) auto-antibodies, presence of anti-Titin antibodies, measure of left ventricular ejection fraction.

The inventors have shown that combining only active thymoma in the patient, presence of cardio-muscular symptoms, left ventricular ejection fraction, voltage (preferably QRS voltage) on presenting electrocardiogram and magnitude of circulating troponin elevation, as markers, makes it possible to obtain a score that is indicative of the risk of MACE related to the myotoxicity. In some embodiments, one can add detection of auto-antibodies against AChR and/or Titin, as a marker included in the test. In some embodiments, presence of auto-antibodies against Titin and/or AChR may be used instead of presence of active thymoma.

In order to use the above markers in the formulas of the methods of the invention, it is necessary to transform the qualitative value of the marker (presence or absence of the phenotype associated with the markers) into a quantitative numerical value. Although various numerical qualitative values could be chosen, and in order to exemplify the methods herein disclosed, the examples show use of discrete numerical values, that depends on the presence and/or the magnitude of the makers.

In particular, one can chose, as numerical values:
- + 2 in the presence of active thymoma, 0 in the absence of active thymoma
- + 1 in the presence of cardio-muscular symptoms, 0 in the absence of cardio-muscular symptoms
- +1 if left ventricular ejection fraction < 50%, 0 if left ventricular ejection fraction ≥ 50%
- + 1 if presence of low voltage (defined by a voltage value ≤ 0.5mV of the sum of the absolute values of the maximal values of R wave in V5 or V6 and of S wave in V1) on presenting electrocardiogram, 0 if absent
- 0 if the cardiac troponin circulating level is below 20-fold (included) of the 99th percentile normal upper limit of the assay as defined by the manufacturer of the assay, + 1 if troponin level is between 20-fold (not included) and 200-fold (included) of the 99th percentile normal upper limit of the assay, + 2 if troponin level is between 200-fold (not included) and 2000-fold (included), + 3 if troponin level is above 2000-fold (not included) of the 99th percentile normal upper limit of the assay
- 0 if anti-AChR (acetylcholine receptor) auto-antibodies are indetectable, +1 if presence of anti-AChR auto-antibodies
- 0 if anti-Titin auto-antibodies are indetectable, +1 if presence of anti-Titin auto-antibodies.

In the preferred embodiment, the cardiac troponin that is used is the cardiac troponin T. However, it is also possible to use the value of the cardiac troponin I if the one of the cardiac troponin T is not available.

The ULN (99^{th} percentile normal upper limit of the assay) may vary according to the manufacture assays. As an illustration, one can cite the following ULN.

**Table 1. ULN for Contemporary Cardiac Troponin I and T Assay Analytical Characteristics Designated by Manufacturer**

| **Company / Manufacturer** | **Troponin type** | **ULN (µg/L)** |
|---|---|---|
| Abbott /ARCHITECT i systems/ ARCHITECT STAT Troponin-I | I | Overall: 0.028 |
| | | F: 0.013 |
| | | M: 0.033 |
| Beckman/Access 2 /AccuTnl+3, US | I | 0.02 |
| Beckman/ Dxl / AccuTnl+3, US | I | <0.03 |
| Beckman/Access 2 /AccuTnl+3, OUS | I | 0.04 |
| Beckman/Dxl/ AccuTnl+3, OUS | I | 0.04 |
| Ortho/VITROS/ Immunodiagnostic Troponin I ES | I | 0.034 |
| Radiometer AQT90 FLEX Tnl | I | 0.023 |
| Radiometer AQT90 FLEX TnT | T | 0.017 |
| Roche cobas e411*/ Roche E170/ cobas e601/602 /co bas e801 cTnl (18 min & STAT) *STAT only | I | 0.16 |
| Roche cobas e411*/ Roche E170/ cobas e601/602 cTnl (18 min & STAT) *STAT only | I | <0.3 |
| Roche cobas e411/ Roche E170/ cobas e601/602 cTnT (18 min STAT) | T | <0.010 |
| Siemens Atellica Tnl-Ultra# | I | 0.020 |
| Siemens ADVIA Centaur Systems Tnl-Ultra# | I | 0.040 |
| Siemens Dimension Vista Systems LOCI CTNI# | I | 0.045 |
| Siemens Dimension EXL Systems LOCI TNI# | I | 0.056 |
| Tosoh AIA cTnl 3rd Gen | I | 0.031 |

**Table 2. ULN for High-Sensitivity Cardiac Troponin I and T Assay Analytical Characteristics Designated by Manufacturer**

| **Company** | **Troponin type** | **ULN (ng/L)** | |
|---|---|---|---|
| Abbott/Alinity i systems/ Alinity i STAT High Sensitive Troponin-I; commercial- OUS | I | Overall: | |
| | | 26.2 | |
| | | F: 15.6 | M: 34.2 |
| Abbott/ ARCHITECT i systems/ ARCHITECT STAT High Sensitive Troponin-I; commercial-US | I | Overall: 28 | |
| | | F: 17 | M: 35 |
| Abbott/ ARCHITECT i systems/ ARCHITECT STAT High Sensitive Troponin-I; commercial | I | Overall: 26.2 | |
| | | F: 15.6 | M: 34.2 |
| Beckman Coulter/ Access 2, Dxl/Access hsTnI; commercial - OUS | I | Overall: 17.5 | |
| | | F: 11.6 | M: 19.8 |
| Beckman Coulter/ Access 2, /Access hsTnI; commercial - U.S.: LiHep plasma | I | Overall: 17.5 | |
| | | F: 11.6 | M: 19.8 |
| Beckman Coulter/ Access 2, /Access hsTnI; commercial - U.S.: Serum | I | Overall: 18.2 | |
| | | F: 11.8 | M: 19.7 |
| Beckman Coulter/ Dxl, Access hsTnI; commercial - U.S.: LiHep plasma | I | Overall: 17.9 | |
| | | F: 14.9 | M: 19.8 |
| Beckman Coulter/ Dxl, Access hsTnI; commercial - U.S.: Serum | I | Overall: 18.1 | |
| | | F: 13.6 | M: 19.8 |
| bioMérieux VIDAS High Sensitive Troponin I; commercial | I | Overall: 19 | |
| | | F: 11 | M: 25 |
| ET Healthcare Pylon hsTnI assay; China FDA approved | I | Overall: 27 | |
| | | F: 21 | M: 27 |
| ET Healthcare Pylon hsTnT; research | T | Overall: 13 | |
| | | F: 13 | M: 14 |
| Fujirebio | I | Overall: 28.6 | |
| | | F: 22.4 | M: 32.9 |
| | | Serum Overall: 26.9 | |
| Lumi pulse G G1200 and G600II hsTnI | | F: 21.4 | M: 29.4 |
| | | Li-Hep plasma | |
| | | Overall: 29.6 | |
| | | F: 27.8 | M: 32.8 |
| LSI Medience PATHFAST cTnI; commercial | I | Overall: 15.46 | |
| | | M: 16.91 | F: 11.46 |
| LSI Medience PATHFAST hs-cTnI /PATHFAST cTnI-II | I | Overall: 27.9 | |
| | | F: 20.3 | M: 29.7 |
| Ortho/ VITROS/ hsTroponin I; commercial | I | Serum Overall: 11 | |
| | | F: 9 | M: 12 |
| | | LiHep Plasma | |
| | | Overall: 11 | |
| | | F: 9 | M: 13 |
| Quidel/Alere TriageTrue hs-cTnI | I | Overall: 20.5 | |
| | | F: 14.4 | M: 25.7 |
| Roche/ cobas e601, e602, e411/cTnT-hs 18-min; commercial | T | Overall:14 | |
| | | F: 9 | M:17 |
| Roche/ cobas e601, e602, e411/ cTnT-hs STAT; commercial | T | Overall: 14 | |
| | | F: 9 | M: 17 |
| Roche/ cobas e801/ e402 cTnT-hs 18-min and 9-min; commercial | T | Overall: 14 | |
| | | F: 9 | M: 17 |
| Roche/ cobas e601, e602, e411 cTnT Gen 5 STAT; commercial | T | Overall: 19 | |
| | | F: 14 | M: 22 |
| Roche / co bas e801/ cTnT Gen 5 - 9 min; commercial | T | Overall: 19 | |
| | | F: 14 | M: 22 |
| Siemens ATELLICA High-Sensitivity TnI (TnIH), US & OUS; commercial# | I | Overall: 45.4 | |
| | | F: 38.6 | M: 53.5 |
| Siemens ATELLICA VTLi hs-cTnl# | I | Overall: 22.9 | |
| | | F: 18.5 | M: 27.1 |
| Siemens ADVIA Centaur XP/ XPT/CP High-Sensitivity TnI (TNIH), US & OUS; commercial*# | I | Overall: 46.5 | |
| | | F: 39.6 | M: 58.0 |
| Siemens Dimension VISTA High Sensitivity TnI (TNIH), OUS; commercial# | I | Overall: 58.9 | |
| | | F: 53.7 | M: 78.5 |
| Siemens Dimension ExL High Sensitivity TnI (TNIH), OUS; commercial# | I | Overall: 60.4 | |
| | | F: 51.4 | M: 76.2 |
| Tosoh CL AIA- PACK cTnI; commercial | I | Overall: < 24 | |
| | | (Asian) < 31 | |
| | | (Caucasian) | |

When using the above values, one can use, as the mathematical function, the sum of the values allocated for each marker.

When such function is used, it is possible to consider that the patient will not have a 30-day major cardio-myotoxic events (MACE) if the result is zero (0) (or that the risk is almost null), and hence that the patient does not need specific treatment, beyond ICI temporary or permanent withhold.

When the score is 1 or higher, the patient is considered to be at risk, and is eligible to a treatment to reduce the irAE, so that the death risk is lowered (and would even be eliminated). Such treatments are disclosed below.

It is to be noted that the value "0" would be allocated to a patient with only (within the elements assessed to establish the predictive score described herein) a troponin level up to 20-fold the ULN level (included). Provided that the other markers are also at "0", the eventual score of the patient would be "0" (using the sum of all values), and the patient would be considered as having no risk of MACE. This finding is unexpected as a troponin level higher than the ULN would be considered as pathologic and dangerous for the patient, and hence trigger immunosuppressive treatment, as currently recommended (ESC Guidelines on cardio-oncology, European Society of Cardiology, 2022). The findings therein show that there is no need to provide treatment to the patient in presence of such troponin value (higher that the ULN and up to 20-fold the ULN, included), and absence of the other clinical sign, as the patient is not to present MACE.

The function described in the examples is illustrative of the methods herein disclosed.

Other functions can be obtained by:
a) evaluating the occurrence of MACE within 30 days in a cohort of patients treated with ICI and for which myotoxicity has been detected, wherein the values or presence of biochemical or clinical markers are known for the patients, and wherein a numerical value has been assigned to the qualitative markers.
b) identifying by unidimensional analysis, the markers for which the values differ significantly between the groups of patients having had 30-days major cardio-myotoxic events or not,
c) performing a regression analysis (generally a logistic regression) to assess and ponder the independent discriminative value of the markers identified in step b) for the occurrence of 30-days major cardio-myotoxic events
d) thereby obtaining a mathematical function, by combination of these identified independent factors, which makes it possible to assess the risk of occurrence of such 30-days major cardio-myotoxic events (MACE).

This method makes it possible to identify the markers that differ between the two groups and to select the markers that can be used in mathematical function to perform the methods herein disclosed.

The method herein disclosed makes it possible to determine whether a patient has an increased risk of having a major cardio-myotoxic event within the following 30 days.

The method herein disclosed (determining increased risk of occurrence of a 30-days major cardio-myotoxic event for the patient) can also be used for determining whether the patient needs to receive an immunosuppressive treatment to reduce the risk of occurrence of such major cardio-myotoxic event, and related death. Indeed, the risk can evolve over time and increase (for instance if administration of the ICI is maintained as before the performance of the method) or decrease (for instance, if administration of the ICI is stopped and/or if appropriate immunosuppressive treatment is provided to the patient, as described below).

The above method can be computer-implemented. In this embodiment, it is possible to perform a method for determining whether a patient has an increased risk of having a major cardio-myotoxic event in the following 30 days, comprising
a) requiring a sender (who could be a healthcare professional, or the patient himself) to identify himself within a public or private network,
b) requiring the sender to fill in information related to the patient, and assigning a specific identifier to the patient in a database;
c) receiving information pertaining to clinical and biochemical markers of the patient, and optionally to the age and gender of the patient, wherein the sender is in a remote place and wherein the values are received in a secure manner, and storing the values in the database, associated with the specific identifier of the patient, allocating a numerical value for each marker according to reference information, and storing the numerical values in a database, wherein storage is associated with the specific identifier of the patient
d) combining the numerical values through a mathematical function, and obtaining an end-value, wherein the end-value is indicative of an increased risk of MACE, and eventually cardiomyotoxicity-related death
e) storing the end-value in a database associated with the specific identifier of the patient and preferably the date and time of identification of the sender, thereby obtaining a database where the information related to the patient is present and associated with the specific identifier,
f) sending the nature of the risk to the sender.

In particular, the information received in c) can also include the name of the kit used to measure troponin, so that the ULN is automatically recovered from a specific database, and used to allocate the numerical value to the troponin marker.

The ECG can be sent in a digital or analogic format, with a processor automatically calculating QRS voltage.

The echocardiography or cardiac MRI can be sent in a digital format, with a processor automatically calculating left ventricular ejection fraction.

The Chest scanner can be sent in a digital format, with a processor automatically evaluating the presence of an active thymoma or not.

In a first step, a sender is required to identify himself to a server within a public or private network. It is preferred when the network is a private network, and when the connection to the server is encrypted. Identification of the sender can be performed using login and password, preferably through strong identification processes (such as one-time password, or using biometric or smart card identification). The sender may be a health-care professional (e.g. physician, pharmacist or a paramedic involved in the care of the patient), or the patient himself or a trusted person selected by the patient.

Upon login to the server, the sender is required to fill-in information related to the patient (such as name, sex, birth date, height, weight, social security identification number, cancer type, ICI type, ICI start date, symptoms, AchR and anti-titin positivity known prior to ICI start) and a specific identifier is assigned to the patient in a database. If the database does not contain any entry relating to the patient, one entry is created. If an entry already exists (using the social security identification number allows ensuring the unicity of entry), such entry is activated.

The server shall then receive information pertaining to the clinical, biochemical and/or radiological markers for the patient (as indicated above), and store such information in a database, associated with the specific identifier of the patient, so that such data is uniquely associated to the patient. Exchanges between the sender's device and the server are very advantageously performed in a secure manner (encrypted exchanges). Age and sex of the patient are to be sent or may be calculated using the information related to the patient. A processor can assign a numerical value for each marker, depending on the information or numerical data received and on references that were preloaded on the server, and store the numerical values in a database associated with the specific identifier of the patient. The numerical values are then combined through a function as herein disclosed and an end-value is obtained, which can be compared to reference values, so that the end-value provides the nature of the 30-days risk of having a major cardio-myotoxic event. As indicated above, the processor may also recover the adequate troponin ULN from a specific database, when the name of the kit used to measure troponin is provided. The processor may also automatically calculate the QRS voltage from an ECG, the left ventricular ejection fraction from an echocardiography or a cardiac MRI, the presence or not of active thymoma from a chest scanner, received from the sender (in digital or analogic format).

It is to be noted that the sender is generally in a remote place different from where the server is located. It is also envisaged that the information is recovered by the server, using information provided by the sender. For instance, the server may open a connection with the server of the laboratory having measured the biochemical markers and/or performed the ECG or any other exam, using identifiers provided by the sender, to upload the required information.

The information pertaining to the comparison of the end-value to the reference values and/or of the nature of the risk can then be stored in a database associated with the identifier specific of the patient and is preferably date and time-stamped. Indeed, since the method may be performed on a regular basis, it may be interesting to keep a record of the results obtained overtime. Thereby, a database is obtained, containing identification related to the patient (patient's specific identifier), associated with the clinical data, and comparison information.

The nature of the risk of the patient, determined according to the methods herein disclosed can then transmitted to the sender.

The invention also relates to a device and/or a computer for implementing the methods herein disclosed. Such device shall comprise processors for assigning a numerical value to the information associated with the patient, combining the numerical value through a function to obtain an end-value, comparing the end-value to reference values and store the data in specific databases.

The device can present one or more of the following, in either combination:
- Operate within a private or public network
- Require the sender to identify himself before sending the inputs
- Receive the inputs (information pertaining to the patient as disclosed above) from a sender that is in a remote place (i.e. they are sent to the device comprising the processor from a different location that where the device is located) in a secure manner
- Assign a numerical value according to the information received for each marker
- Combine the numerical values to obtain an end-value
- Determine the nature of the risk for the patient
- Send the output (end-value and/or risk) to the sender of the inputs
- Store the output in a database (possibly with a unique identifier, making it possible to assign inputs, outputs to this identifier)
- Provides the nature of the risk with further information (such as sensitivity and/or specificity and/or positive predictive value and/or negative predictive value)

The invention also relates to a computer-implemented method for determining the risk of a 30-day major cardio-myotoxic event in a subject, comprising
a) Receiving inputs from a sender, wherein the inputs are information pertaining to clinical, biochemical, and/or radiological markers obtained for the subject, optionally as well as the information pertaining to the age, height, weight, and sex of the subject
b) Performing the method as disclosed herein, to obtain an output (end-result) for the combination of numerical values assigned for each marker from the information received in (a),
c) optionally comparing the output to a reference value and determining the risk of a 30-day major cardio-myotoxic event for the patient
d) Sending the output to the sender and the risk of occurrence of risk of a 30-day major cardio-myotoxic event, if determined, and optionally further information (such as sensitivity and/or specificity and/or positive predictive value and/or negative predictive value)
e) Optionally storing the inputs and the output in a database (preferably with a unique identifier associated with the patient and/or with the sender, making it possible to assign inputs, outputs to this identifier)

The invention also relates to a method for obtaining the information pertaining to the risk of a 30-days major cardio-myotoxic events, comprising
a) Sending inputs to a remote server that is in a remote place (i.e. they are sent to the first server that is in a different location than the location of the sender), preferably in a secure (encrypted) manner, wherein the inputs are information pertaining to clinical or biochemical markers obtained for the subject, optionally as well as the information pertaining to the age, height, weight and sex of the subject
b) Having the remote server combine numerical values assigned for each marker from the information received in (a) in a function, according to the methods herein disclosed, and obtain an output (end-value), optionally normalized
c) optionally having the remote server compare the output to a reference value and determining the risk of occurrence of a 30-day major cardio-myotoxic event for the subject
d) Optionally having the remote server store the inputs and the output in a database (possibly with a unique identifier associated with the subject and/or with the sender, making it possible to assign inputs, outputs to this identifier)
e) receiving the output from the server and/or the information pertaining to the risk of occurrence of a 30-days major cardio-myotoxic event (when determined) and optionally further information (such as sensitivity and/or specificity and/or positive predictive value and/or negative predictive value).

Is also foreseen a non-transitory computer readable storage medium, having stored therein a computer program comprising program instructions, the computer program being loadable into a data-processing unit and adapted to cause the data-processing unit to carry out a method for calculating the 30-days risk of having a major cardio-myotoxic event from the markers of a patient as disclosed through a method as disclosed above, when the computer program is run by the data-processing device.

In another embodiment, the invention relates to a microprocessor comprising a computer algorithm to perform a method for calculating the 30-days risk of having a major cardio-myotoxic event from the markers of a patient as disclosed through a method as disclosed above.

The invention also relates to the use of the functions, devices and/or microprocessors in order to assess the 30-days risk of having a major cardio-myotoxic event by use of the methods herein disclosed.

In the above method, clinical information preferably includes ECG, cardiac imaging presence of cardiac symptoms, presence of active thymoma, and biochemical information preferably includes level of cardiac troponin, preferably cardiac troponin T, and AchR and anti-titin auto-antibodies.

The patients identified by the methods herein disclosed may be treated if they are identified as having a risk of major myotoxicity-related adverse effect.

In particular, one can use high-dose corticosteroids. Corticosteroids doses range from bolus of 0.5-2mg/kg/day of prednisone up to 1g/day methylprednisolone, depending on severity of clinical presentation. If symptoms and laboratory findings do not improve or worsen with steroids, other immunosuppressive drugs may be also used. One can cite antimetabolites (such as mycophenolate mofetil, azathioprine, methotrexate), anti-calcineurin (also designated as calcineurin inhibitors) (ciclosporin, tacrolimus), mTOR inhibitors (sirolimus, temsirolimus, everolimus), anti-thymoglubin, intravenous immunoglobulin, interleukin-6 inhibitors (tocilizumab, siltuximab, sarilumab, sirukumab, olokizumab, clazakizumab), interleukin-1 pathway inhibitors (anakinra, rilonacept, canakinumab), TNF-o inhibitors, or anti CD28. Other compounds that can be used also include basiliximab (chimeric mouse-human monoclonal antibody) or daclizumab (both binding to the α chain (CD25) of the IL-2 receptor of T cells), tocilizumab, also known as atlizumab, humanized monoclonal antibody against the interleukin-6 receptor (IL-6R), and alemtuzumab (monoclonal antibody that binds to CD52). In case of associated myositis and/or myasthenia gravis, intravenous immunoglobulin or plasmapheresis can be considered when presentations are severe and/or corticosteroid-resistant.

It is however preferred to use a CTLA4 agonist for treating or preventing worsening of the ICI-induced myotoxicity, notably when the ICI is an anti-PD1, an anti PDL-1, an anti CTLA4 or an anti-LAG3.

Among CTLA4 agonist, one can cite belatacept and abatacept. It is preferred to use abatacept for the treatment of ICI-induced myotoxicity in a patient, when the patient presents an identified risk (i.e. the patient is not classified in the "no or low risk" class, i.e. risk-score = 0). When using the specific markers as disclosed above, the numerical values associated therewith and the sum of the numerical value as the function, such patients (at risk) are the ones for whom the obtained score is higher than or equal to 1 (one). For patients with a score equal to 1, abatacept can be used alone, potentially with corticosteroids. Alternatively, for these patients, corticosteroids can be used alone, as currently recommended.

Dosage of abatacept may be adjusted by checking the saturation rate of CD86 receptors on circulating monocytes (CD86RO), and increasing the abatacept dosage if the rate is below 60%, preferably at least reaching 80% within 72 hours of each abatacept administration until reaching a cardiac troponin-T circulating level below 20 ULN and resolution of MACE. CD86RO can be assessed by cytometry: the basal level of CD86 receptors on circulating monocytes (or B-cells or other circulating antigen presenting cells, in another embodiment) expression is measured before introduction of the treatment with abatacept or any other CTLA4 agonist, or of novel administration of abatacept (at least 2 months off any CTL4 agonist). After administration of abatacept, the level of CD86 receptors expression is again measured and compared with the basal level, thus indicating the actual saturation level (percentage of the CD86 receptors occupied by abatacept).

It is also possible to use a JAK inhibitor for treating patients for the ICI-induced myotoxicity. In particular, one can use a JAK inhibitor, when the end value obtained when applying the identification method to the patient is higher than or equal to 1 (using the markers, numerical values associated therewith and function as disclosed above). The JAK inhibitor can be used alone (notably for patients with an end value equal to one) or with corticosteroids.

By JAK inhibitor, it is intended to designate a molecule that inhibits the activity of one or more of the Janus kinase family of enzymes, thereby interfering with the JAK-STAT signaling pathway. Some examples of effect of the JAK/STAT signaling pathway are production of colony-stimulating factor, prolactin, growth hormone, and many cytokines. Janus kinases (or JAKs) is an intracellular, non-receptor tyrosine kinase family consisting of four different subtypes, namely JAK1, JAK2, JAK3, and TYK2. JAK1, JAK2, and TYK2 are ubiquitously expressed, while JAK3 is mainly localized in hematopoietic cells.

In an embodiment, the JAK inhibitor is a TYK2 inhibitor.

In an embodiment, the JAK inhibitor is a JAK3 inhibitor.

In an embodiment, the JAK inhibitor is a JAK2 inhibitor. One can cite fedratinib (N-tert-Butyl-3-{5-methyl-2-[4-(2-pyrrolidin-1-yl-ethoxy)-phenylamino]-pyrimidin-4-ylamino}-benzenesulfonamide), gandotinib (3-(4-Chloro-2-fluorobenzyl)-2-methyl-N-(5-methyl-1H-pyrazol-3-yl)-8-(morpholinomethyl)imidazo[1,2-b]pyridazin-6-amine), lestaurtinib ((5S,6S,8R)-6-Hydroxy-6-(hydroxymethyl)-5-methyl-7,8,14,15-tetrahydro-5H-16-oxa-4b,8a, 14-triaza-5,8-methanodibenzo[b,h] cycloocta[jkl]cyclopenta[e]-as-indacen-13(6H)-one), or pacritinib.

In an embodiment, the JAK inhibitor is a JAK1 inhibitor. One can cite, in particular oclacitinib (N-Methyl{trans-4-[methyl(7H-pyrrolo[2,3-d]pyrimidin- 4-yl)amino] cyclohexyl}methanesulfonamide), upadacitinib ((3S,4R)-3-Ethyl-4-(3H-imidazo[1,2-a]pyrrolo[2,3-e]pyrazin-8-yl)-N-(2,2,2-trifluoroethyl)pyrrolidine-1-carboxamide), filgotinib (N-[5-[4-[(1,1-Dioxo-1,4-thiazinan-4-yl)methyl]phenyl]-[1,2,4]triazolo [1,5-a]pyridin-2-yl]cyclopropanecarboxamide), abrocitinib (N-(cis-3-(Methyl(7H-pyrrolo(2,3-d)pyrimidin-4-yl)amino)cyclobutyl)propane-1-sulfonamide).

In an embodiment, the JAK inhibitor is a JAK1/JAK2 inhibitor. One can cite, in particular, ruxolitinib ((3R)-3-Cyclopentyl-3-[4-(7H-pyrrolo[2,3-d]pyrimidin-4-yl)pyrazol-1-yl]propanenitrile), described in WO2007070514, baricitinib (2-[1-Ethylsulfonyl-3-[4-(7H-pyrrolo[2,3-d]pyrimidin-4-yl)pyrazol-1-yl]azetidin-3-yl]acetonitrile) and momelotinib (N-(cyanomethyl)-4-{2-[4-(morpholin-4-yl)anilino]pyrimidin-4-yl}benzamide).

It is preferred when the JAK inhibitor is selected from the group consisting of ruxolitinib, tofacitinib, oclacitinib, baricitinib, peficitinib, fedratinib, upadacitinib, filgotinib, cerdulatinib, gandotinib, lestaurtinib, momelotinib, pacritinib and abrocitinib.

Tofacitinib is also of particular interest.

Baricitinib is also of particular interest.

JAK1/JAK2 inhibitor are particularly preferred, in particular ruxolitinib, which presents selectivity for subtypes JAK1 and JAK2.

It is advisable, notably for patients with an end value equal to or higher than 2, to use a combination of such molecules. Notably, one would use both the CTLA4 agonist (preferably abatacept) and the JAK inhibitor, preferably but not necessarily together with corticosteroids, in combination for treating the patients.

A preferred treatment would include abatacept, ruxolitinib and a corticosteroid. In some embodiments, the treatment includes abatacept and ruxolitinib, without the use of a corticosteroid. In these latter embodiments, the initial dosage of abatacept and/or ruxolitinib may be higher than the dosage used when corticosteroids are also administered.

The treatment could follow the following regimen:
- treatment is initiated at Day 0 by a single administration of abatacept, and a daily administration of the JAK inhibitor and the corticosteroid
- at Day 5±2, clinical situation of the patient is evaluated (notably looking for worsening (see below) or seeking for MACE status appearance or resolution) and treatment comprises another single administration of abatacept, and adjusting (maintaining, increasing or decreasing) treatment with the JAK inhibitor and/or corticosteroid treatment according to respectively stabilization, worsening or improvement of the clinical situation of the patient
- at Day 14±2, clinical situation of the patient is evaluated and treatment comprises another single administration of abatacept, and adjusting (maintaining, increasing or decreasing/weaning) treatment with the JAK inhibitor and/or corticosteroid treatment according to respectively stabilization, worsening or improvement of the clinical situation of the patient
- from Day 14±2 to D21±2, increasing corticosteroid and/or JAK inhibitor dosage in case of worsening of the clinical situation of the patient
- from Day 21±2 to Day 120±2, performing assessment of the clinico-biological situation of the patient and step by step lowering until weaning of corticosteroid and JAK inhibitor dosage (every 5 to 15 days) in case of improvement; maintaining corticosteroid and JAK inhibitor dosage and administering abatacept in case of worsening and; lowering corticosteroid dosage, maintaining JAK inhibitor dosage and administering abatacept in case of stability. The abatacept dosage is preferably adjusted so as to reach over 80% of saturation of CD86RO within each new abatacept administration.

One considers that there is clinical worsening in case of appearance of at least one new severity criteria:
- ventricular tachycardias, and/or high-grade conductive disorders,
- and/or symptomatic heart failure requiring intravenous treatment (including diuretics) or mechanical support
- and/or respiratory muscle failure leading to hypercapnia or requiring mechanical ventilation,
- and/or dysphagia requiring nasogastric tube or parenteral feeding,
- and/or biological worsening with daily doubling of circulating troponin (preferentially -T, or -I otherwise) 3 days in a row

One considers that there is clinical improvement when there is no appearance of new severity criteria and a drop in the Troponin-T level (50% from the peak level observed within the prior 15 days, or reaching a level ≤32 times the ULN).

If the situation is not worsened nor improved as indicated above, clinical situation is considered as stable.

The treatment can thus be started with the following regimen:
- Abatacept is administered at starting dosage of 5 to 25 mg/kg IV at day 1
- Prednisone is administered at a starting dosage of 0.1 to 1 mg/kg per day *per* os
- Ruxolitinib is administered at a starting dosage of 5 to 30 mg twice a day *per os.*

Such treatment is provided at least once and is preferably maintained for at least three or at least five days. The treatment can be maintained up to 90 days, depending on the evolution of the cardiomyotoxicity for the patient.

In other embodiments and according to the physician's evaluation of the clinical state of the patient and of the result of the bio-radiological tests, these drugs can be used alone, in monotherapy, in bitherapy or all three drugs in combination. Preferably, all three drugs are used in combination for patients with a score higher or equal to 2. For patients with a score equal to one, mono or biotherapies are possible (within CTLA4 agonists, or JAK inhibitors, or corticosteroids). It is reminded that patients with a score equal to 0 do not need to be treated by immunosuppressant drugs (except for suspension of ICI-treatment).

Preferred dosages and regimen are:
- for starting the treatment
   i. abatacept: single injection of between 5 and 25 mg/kg, generally about 20mg/kg (intravenously)
   ii. ruxolitinib: about 15 mg (5-30 mg) twice a day *per os*, or enterally if oral intake is not possible
   iii. corticosteroid (prednisone): about 1 mg/kg/day (0.1-1mg/kg/day) *per os*, or intravenously or enterally if oral intake is not possible, generally in the morning
- at day 5 (±2) (second injection of abatacept)
   i. abatacept: single injection of between 5 and 25 mg/kg, generally about 20mg/kg (intravenously)
   ii. ruxolitinib: (5-30 mg) twice a day *per os, eventually* lowering the dosage if important bettering (reaching a cardiac troponin-T circulating level below 20 ULN)
   iii. corticosteroid: dosage increase (up to 2 mg/kg/day) and possibly introduction of another immunosuppressive drug (Mycophenolate Mofetil: 3g/day) if worsening, maintaining the starting dosage if stability, lowering or weaning the dosage if bettering
- at day 14 (±2) (third injection of abatacept)
   i. abatacept: single injection of between 5 and 25 mg/kg, generally about 20mg/kg (intravenously)
   ii. ruxolitinib: 5-15mg twice a day *per os* if stability, 5mg twice a day *per os* if bettering, or completed weaning if reaching a cardiac troponin-T circulating level below 20 ULN
   iii. corticosteroid: maintaining the same dosage than day 5 (±2) if stability, lowering or weaning the dosage if bettering

Follow-up is then performed between day 14 (±2) and day 21 (±2), and immunosuppressive drugs (corticosteroids, mycophenolate mofetil) or ruxolitinib are increased (if they had been lowered) or introduced (mycophenolate mofetil) in case of worsening. In case of stability or bettering, no treatment change.

From day 21 (±2), following the above treatment, the clinical condition of the patient should be stabilized and the risk of a major cardiomyotoxicity adverse effect should be minimized. It is thus advisable to decrease the dosage of corticosteroids and of ruxolitinib (if not already weaned) every 5 to 15 days in case of improvement. In case of stability or worsening (reevaluated every 5 to 15 days until complete immunossupressant weaning), corticosteroid and JAK inhibitor dosage should be maintaining and complementary administration of abatacept (with dosage adjusted on CD86RO before each new abatacept administration) should be done.

Such treatment, with such dosages, can be performed for patients identified as at risk of MACE when the methods herein disclosed have been performed. They can also be performed for any patient that would consult with ICI-induced myotoxicity notably polymyositis potentially affecting the heart (myocarditis) and/or peripheral muscles (myositis) potentially mimicking myasthenia-gravis, even if no measure of risk has been done. In this embodiment, it is acknowledged that patients with no risk would also receive the treatment. Although not the optimal way of treating patients, this embodiment would still be performed in the impossibility of having a risk stratification of the patients.

A method for treating a patient under Immune Checkpoint Inhibitor treatment (or which has received such treatment) and presenting an ICI induced myotoxicity with an increased risk of MACE and cardiomyotoxicity-related death, comprising administration of the CTLA4 agonist (notably abatacept), JAK inhibitor and corticosteroid, as disclosed above, notably when the patient is identified as being at risk of MACE, can thus be implemented, notably using the above-dosages and follow-up of the clinical situation of the patient.

### DESCRIPTION OF THE FIGURES

**Figure 1****:** Cardiac workup and clinical event rate. Time from first ICI dose to onset of myocarditis (A), and event-curve of first major cardio-myotoxic event at 30-days (B).
**Figure** 2: Risk score for major cardio-myotoxic events. Components of the risk score of major cardio-myotoxic events at 30-days (A). Distribution of patients according to risk score (B). Cumulative incidence of major cardio-myotoxic events according to for risk score level (C). Cumulative incidence of components of the primary composite outcome of severe cardio-myotoxic events (D).
**Figure 3****:** Validation cohort risk score distribution and cardio-myotoxic event rate. Risk score distribution for Sorbonne University validation cohort (A). Cumulative incidence of major cardio-myotoxic events according to the risk score level in the Sorbonne University validation cohort (B).

### EXAMPLES

### Example 1. Summary

**Background:** Immune-checkpoint inhibitors (ICI) are associated with life-threatening myocarditis but milder presentations are increasingly recognized. The same autoimmune process that causes ICI-myocarditis can manifest concurrent generalized myositis, myasthenia-like syndrome, and respiratory muscle failure. Prognostic factors for this "cardiomyotoxicity" are lacking.

**Methods:** A multicenter registry collected data retrospectively from 17 countries between 2014-2023. A multivariable cox regression model (hazard-ratio(HR),[95%confidence-interval]) was used to determine risk factors for the primary composite outcome: severe arrhythmia, heart failure, respiratory muscle failure, and/or cardiomyotoxicity-related death. Covariates included demographics, comorbidities, cardio-muscular symptoms, diagnostics, and treatments. Time-dependent covariates were used and missing data were imputed. A point-based prognostic risk score was derived and externally validated.

**Results:** In 748 patients(67% male, age 23-94), 30-day incidence of the primary composite outcome, cardiomyotoxic death, and overall death were 33%, 13%, and 17% respectively. By multivariable analysis, the primary composite outcome was associated with active thymoma (HR=3.60[1.93-6.72]), presence of cardio-muscular symptoms (HR=2.60 [1.58-4.28]), low-voltage on presenting electrocardiogram (HR for ≤0.5mV versus >1mV=2.08[1.31-3.30]), left ventricular ejection fraction (LVEF) <50% (HR=1.78[1.22-2.60]), and incremental troponin elevation (HR=1.86 [1.44-2.39], 2.99[1.91-4.65], 4.80[2.54-9.08], for 20, 200 and 2000-fold above upper reference limit, respectively). A prognostic risk score developed using these parameters showed good performance; 30-days primary outcome incidence increased gradually from 3.9%(risk-score=0) to 81.3%(risk-score≥4). This risk-score was externally validated in an independent French prospective cohort. This risk score was used prospectively in the external French cohort to identify low risk patients who were managed with no immunosuppression resulting in no cardio-myotoxic events. **Conclusions:** ICI-myocarditis can manifest with high morbidity and mortality. Myocarditis severity is associated with magnitude of troponin, thymoma, low-QRS voltage, depressed LVEF, and cardio-muscular symptoms. A risk-score incorporating these features performed well, was externally validated and was used to personalize treatment.

### Example 2. Methods

### Data Collection

A retrospective registry spanning 127 institutions across 17 countries containing 757 cases of ICI-myocarditis diagnosed by treating clinicians was used as a discovery cohort. Clinical data were locally adjudicated and submitted by participating centers via a HIPPA-compliant REDCap web-based platform (IRB:181337; NCT04294771). Submissions were reviewed and cases were rejected when evidence did not meet Bonaca's diagnostic criteria for myocarditis, a hierarchical definition incorporating the availability and strength of diagnostic data.¹³ Electrocardiograms (ECG) on presentation were independently interpreted by two cardiologists blinded to specific cases who performed quantitative measurements (Sokolow-Lyon Index, PR, and QRS length) and identified qualitative features (methods previously published).^{14,15} The primary outcome, major (or severe) cardio-myotoxic events (MACE), was defined as any of the following: 1) severe heart failure requiring either intravenous medicines or mechanical circulatory support 2) severe arrhythmia defined as sustained ventricular arrythmia, complete heart block, sudden cardiac death, use of atropine or isoproterenol, and/or use of pacemaker/defibrillator 3) respiratory failure due to myositis-related respiratory muscles failure requiring ventilation and/or 4) death due to these conditions (henceforth referred to as cardio-myotoxicities). Respiratory muscle failure was included to reflect contemporary understanding of ICI-myocarditis as part of a generalized skeletal myositis that can impair ventilation.^{5,8,16,17} The study period ended 30 days after clinical presentation with ICI-myocarditis to avoid including unrelated endpoints. Cases were excluded if the primary outcome was met more than 24 hours prior to presentation (n=9), leaving 748 cases for analysis.

One external independent validation ICI-myocarditis cohorts (using the updated Bonaca's criteria) was obtained from Sorbonne University (SU, Paris, France) including a prospective cohort of 30 consecutive cases presenting between April 2023 and February 2024 where the risk score was used to guide treatment; *NCT05454527*)^{5*,*18}*.* This external validation cohort was collected after derivation of a risk-score from the discovery cohort. The same inclusion and exclusion criteria, and outcomes definition from the discovery cohort were applied.

### Statistical Analysis

### Explanatory model

A univariate cox regression model was used to evaluate the association of clinical variables with major cardio-myotoxic events in the discovery cohort. Independent variables spanned past medical history, objective data collected during the ICI-myocarditis evaluation, and immunosuppressant use. Troponin, creatine-kinase, cardio-muscular symptoms, left ventricular ejection fraction (LVEF), and immunosuppressant use were treated as time-dependent covariates to prevent survivorship bias. Troponin and creatine-kinase were divided by the contributing institution's upper limit of normal (based on ULN, 99^{th} percentile upper limit of the normal provided by the manufacturers of the assay used in each institution). When both cardiac troponin-T and troponin-I were collected, cardiac troponin-T was preferentially used.¹⁸ A multivariable explanatory model tested pre-selected variables based upon available evidence and clinical experience. Multivariable analysis was performed after multiple imputation by chained equations of missing data using 50 imputed datasets. Imputed results were merged according to Rubin's rule.²⁰

### Predictive model

Starting from an empty predictive model, a stepwise selection procedure was performed using Schwartz Criteria to select the most impactful variables. The untransformed coefficients of these variables were used to derive a risk score for predicting the primary outcome with one point assigned for a coefficient of 0.5-1, two points for a coefficient of >1-1.5, and three points for a coefficient >1.5. Internal validation was performed with K-fold cross-validation with k-set of 10. External validation was performed on an external validation ICI-myocarditis cohort. For both internal and external validation, Harrell's c-index assessed the risk score's goodness-of-fit using serial models on days 1-3. Confidence intervals were obtained with bootstrap, using up to 1000 bootstrap replicates. Sensitivity analysis was performed with an unimputed dataset as well as a competing risk model to account for death from other causes. Analyses were conducted with R-v.4.2.2 for Windows, and the R-packages survival, mice, boot and rms. A two-sided p-value<0.05 was deemed significant.

### Example 3. Results

### Patient Characteristics and Outcomes

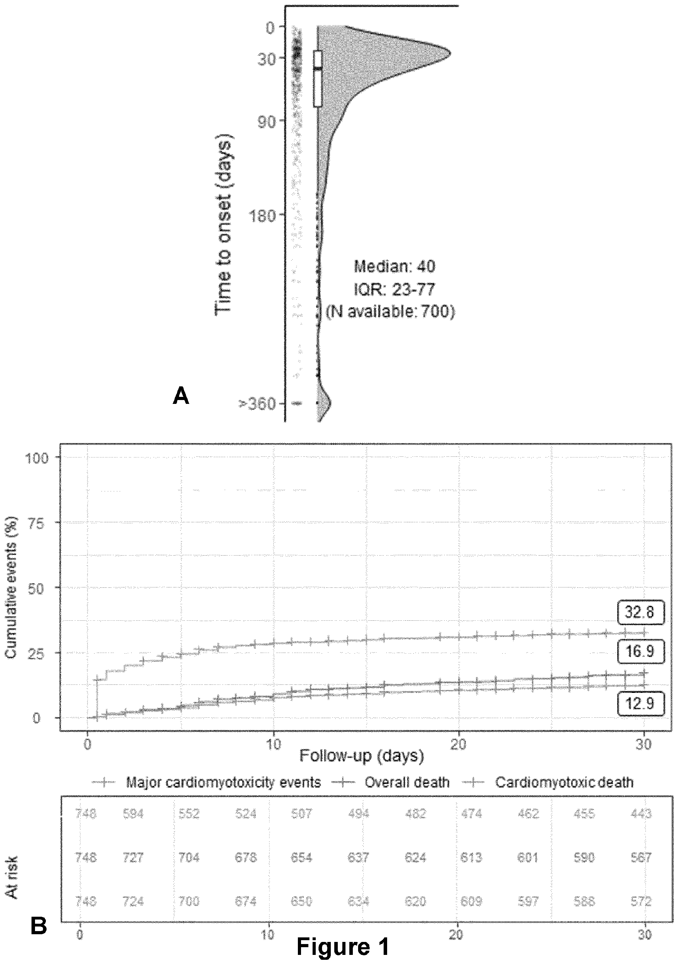

Among 748 cases of ICI-myocarditis, median(interquartile-range) age was 69(61-77) and 33% were female. Median time from first ICI dose to myocarditis presentation was 40 (23-77) days with 21% patients receiving combination ICI and 79% receiving ICI monotherapy (usually anti-PD-1), as seen on Figure 1A. The most common primary cancers were skin (31%), lung (24%), and genitourinary (22%); 3% had a thymoma. Half the cohort was classified as definite myocarditis, and one quarter each as probable and possible. Among those who underwent cardiac MRI (magnetic resonance imaging, n=459), studies were consistent with myocarditis in 64% patients and when endomyocardial biopsy or autopsy was performed(n=290), histopathology was consistent with myocarditis in 67% patients. Patients without supporting MRI or histopathology met myocarditis criteria by other diagnostics; 84% had cardio-muscular symptoms within 72h of presentation, 95% had elevated troponin, 94% had abnormal ECG, 42% had depressed LVEF, and 43% had either concomitant myositis or myasthenia gravis-like syndrome. Corticosteroids were given to 83% of patients and 36% received non-steroidal immunomodulators.

The primary composite outcome of major cardio-myotoxic events occurred in one third of patients within one month of presentation. This included 118/748(16%) patients with heart failure, 143/748(19%) with arrhythmia, 61/748(8%) with respiratory muscle failure, and 92/748(13%) with death due to cardio-myotoxicity. Most cardio-myotoxicity events and related deaths occurred early in the 30-days study period while overall death (17%) was more evenly distributed (Figure 1B for event curves).

### Explanatory Analysis

Multivariable modeling found that major cardio-myotoxic events were associated with active thymoma (HR=3.75[1.82-7.69]), recent ICI initiation (HR for 10 versus 30 days=1.06 [1.01-1.11]), presenting ECG with low QRS-voltage (HR for ≤0.5mV versus >1mV =1.88 [1.15-3.07]), LVEF<50% (HR=1.78 [1.19-2.67]), presence of cardio-muscular symptoms (HR=2.60 [1.57-4.31]) and troponin elevation (HR=1.83 [1.38-2.43], 2.91 [1.76-4.81], 4.63 [2.25-9.53] for 20, 200 and 2000-fold increase over upper reference limit. In this model, notable variables that showed no association with severe cardio-myotoxicity included age, sex, combination ICI therapy, and corticosteroid dose.

Univariate analysis associated major cardio-myotoxic events with several features, some of which did not meet significance upon multivariable analysis. Those included high body mass index (HR=1.10 [1.00-1.21]), cardio-muscular symptoms (HR=3.44 [2.10-5.63]), pathological Q-waves (HR=1.96 [1.34-2.89]), magnitude of QRS prolongation (HR=1.64 [1.18-2.27] for 150ms vs. 90ms), elevated creatine kinase (HR for 50-fold increase over ULN=2.44 [1.77-3.35]), elevated neutrophil-to-lymphocyte ratio (HR=1.03 [1.01-1.04]), and treatment with non-steroidal immunomodulators (HR=1.98 [1.34-2.94]). Similar results were found when multivariable modeling was performed on an unimputed complete dataset. A competing risk analysis correcting for the effect of death due to other causes had overall unchanged findings and effect size compared to the original multivariable model.

### Predictive Analysis and Risk Score

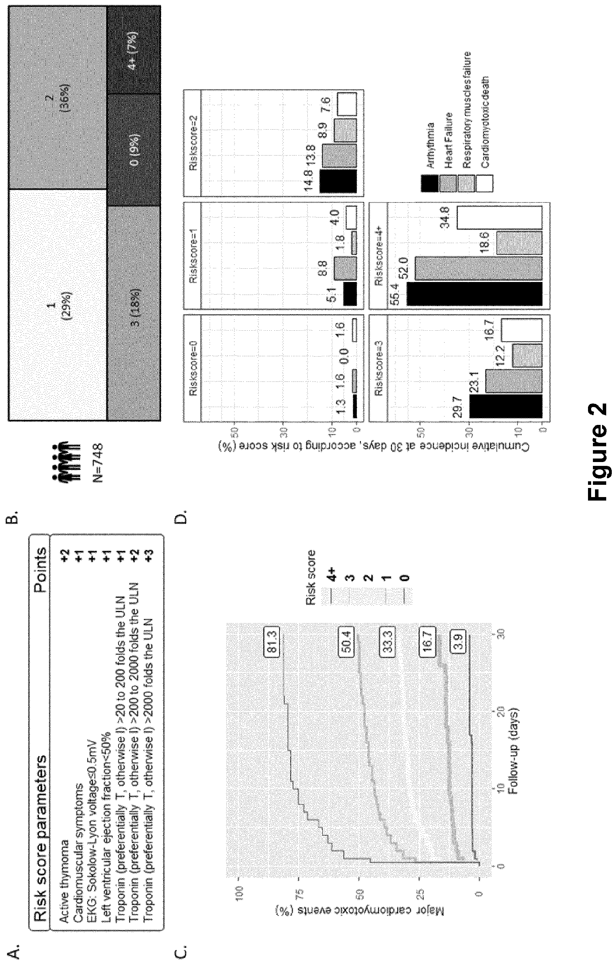

A stepwise variable selection procedure identified the strongest predictors of the composite major cardio-myotoxic event outcome to be active thymoma (HR=3.60 [1.93-6.72]), presence of cardio-muscular symptoms (HR=2.60 [1.58-4.28]), low-voltage on presenting electrocardiogram (HR for ≤0.5mV versus >1mV=2.08 [1.31-3.30]), LVEF<50% (HR=1.78 [1.22-2.60] vs. LVEF≥50%), and elevated troponin (HR=1.86 [1.44-2.39], 2.99 [1.91-4.65], 4.80 [2.54-9.08], for 20, 200 and 2000-fold increase over ULN, respectively). A risk score ranging from 0 to 8 was derived by assigning each predictor a value proportional to its respective regression coefficient: +2 for active thymoma, +1 for cardio-muscular symptoms, +1 for low-voltage, +1 for LVEF<50%, and +1-3 points for degree of troponin elevation (Figure 2.A). Classification of the cohort according to risk score on day of presentation showed 9% with 0 points, 29% with 1 point, 36% with 2 points, 18% with 3 points, and 4% with 4 or more points (Figure 2.B). 30-day cumulative incidence of major cardio-myotoxic events was low at 3.9% for patients with risk score of 0 and increased as risk score increased with 16.7% incidence for 1 point, 33.3% for 2 points, 50.4% for 3 points, and 81.3% for 4 or more points (Figure 2.C). There was a 1.6% cumulative incidence of cardio-myotoxicity related death for a risk score of 0 compared to 34.8% for a score≥4. All components of the primary outcome increased incrementally with higher risk scores (Figure 2.D).

### Risk Score Validation

Internal validation of the risk score showed good performance with a c-index of 0.70 [0.67-0.73] on day one and remained unchanged at 0.71 [0.66-0.75] by day three (Table 4). A score of 0 had a negative predictive value of 97% [90-100] for severe myotoxicity and 98% [91-100] for myotoxicity-related death (Table 3).

**Table 3. Diagnostic properties of the risk score to predict major (severe) cardio-myotoxic events (MACE) at 30 days**

| Risk Score | Sensitivity | Specificity | Positive Predictive Value | Negative Predictive Value |
|---|---|---|---|---|
| Discovery Cohort: International Redcap | | | | |
| 0+ | 1.00 (0.98-1.00) | 0.00 (0.00-0.01) | 0.32 (0.29-0.36) | |
| 1+ | 0.99 (0.97-1.00) | 0.14 (0.11-0.17) | 0.35 (0.32-0.39) | 0.97 (0.90-1.00) |
| 2+ | 0.82 (0.77-0.87) | 0.48 (0.44-0.52) | 0.43 (0.39-0.48) | 0.85 (0.80-0.89) |
| 3+ | 0.46 (0.40-0.53) | 0.84 (0.81-0.87) | 0.59 (0.52-0.65) | 0.77 (0.73-0.80) |
| 4+ | 0.18 (0.14-0.24) | 0.98 (0.96-0.99) | 0.80 (0.68-0.89) | 0.71 (0.68-0.75) |
| 5+ | 0.06 (0.03-0.09) | 1.00 (0.99-1.00) | 0.91 (0.65-0.99) | 0.69 (0.65-0.72) |
| 6+ | 0.01 (0.00-0.04) | 1.00 (0.99-1.00) | 0.96 (0.35-0.99) | 0.68 (0.64-0.71) |

| Validation Cohort: Sorbonne University | | | | |
|---|---|---|---|---|
| 0 | 1.00 (0.66-1.00) | 0.00 (0.00-0.18) | 0.30 (0.17-0.48) | |
| 1 | 1.00 (0.66-1.00) | 0.33 (0.17-0.55) | 0.39 (0.22-0.59) | 1.00 (0.60-1.00) |
| 2 | 0.89 (0.54-1.00) | 0.62 (0.41-0.79) | 0.50 (0.28-0.72) | 0.93 (0.66-1.00) |
| 3 | 0.44 (0.19-0.73) | 0.90 (0.70-0.99) | 0.67 (0.30-0.91) | 0.79 (0.59-0.91) |
| 4 | 0.22 (0.05-0.56) | 0.95 (0.76-1.00) | 0.67 (0.20-0.94) | 0.74 (0.55-0.87) |
| 5 | 0.00 (0.00-0.34) | 1.00 (0.82-1.00) | | 0.70 (0.52-0.83) |
| 6 | 0.00 (0.00-0.34) | 1.00 (0.82-1.00) | | 0.70 (0.52-0.83) |

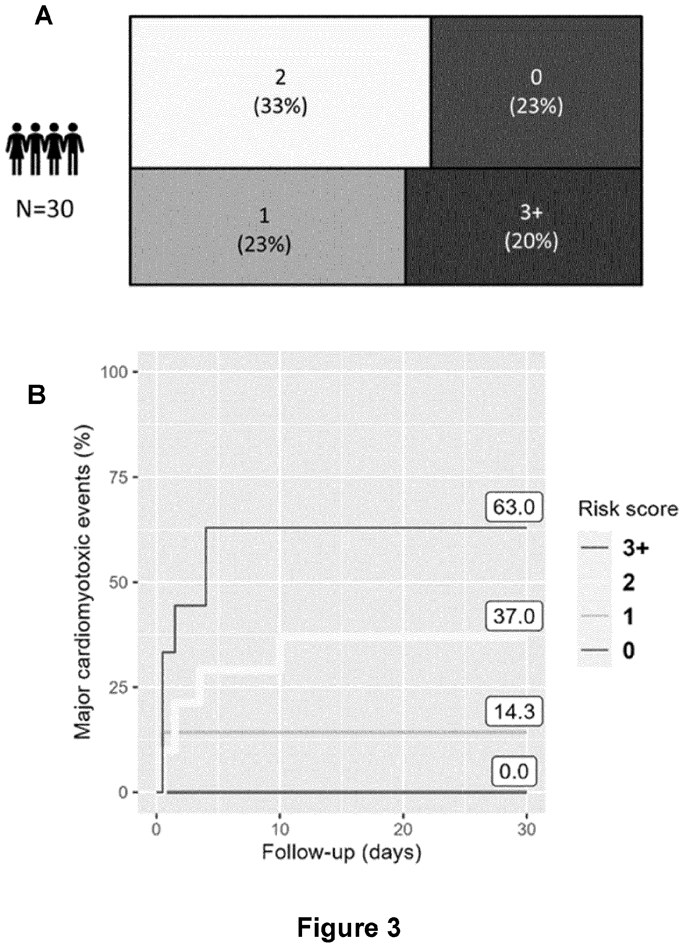

Compared to the derivation cohort, the SU cohort had a higher proportion of score 0 (23%), but scores 1, 2, and 3+ were similar (23%, 33%, and 20%, respectively) to the discovery cohort (Figure 3.A). For risk score 0, 30-days cumulative incidence of major cardio-myotoxic events was 0% in and increased incrementally for score 1 (14.3%), score 2 (37.0%), and was highest for risk score 3+ (63%) (Figure 3.B). In the SU cohort, the risk score had c-index of 0.76 (0.57-0.88) on day 1 and a risk score of 0 had 100% negative predictive value (Table 3, Table 4). In the prospective SU validation cohort, all seven patients with risk score of 0 had ICI withheld but no immunosuppression. There was no death or major cardiomyotoxic events in these seven patients over one month.

**Table 4. Harrel's c-index of risk score for association with major cardio-myotoxic events, when applied on a daily basis to Discovery Cohort and Sorbonne University Validation Cohort.**

| | Discovery cohort | Sorbonne University Validation cohort |
|---|---|---|
| Day 1 | 0.700 (0.667-0.730) | 0.759 (0.566-0.884) |
| Day 2 | 0.711 (0.662-0.755) | 0.803 (0.441-0.925) |
| Day 3 | 0.709 (0.659-0.754) | |

### Example 4. Discussion

ICI-myocarditis manifests with a wide range of severity. This study used a contemporary multicenter registry to follow 748 patients with ICI-myocarditis for one month after presentation, observing 13% mortality from cardio-myotoxicity. ICI-myocarditis was highly morbid with a primary composite outcome of severe heart failure, arrhythmia, respiratory muscle failure, and cardio-myotoxicity-related mortality occurring in 33% of patients. Multivariable analysis showed this primary outcome was associated with elevated troponin, thymoma, cardio-muscular symptoms, LVEF<50%, and ECG with low voltage. To avoid over-fitting and favor generalization, a simplified risk score was generated from these features which predicted adverse outcomes with good accuracy and very high negative predictive value. The risk score was externally validated in a prospective cohort of 30 patients at Sorbonne University (Paris, France). In the prospective cohort, immunosuppressants were withheld in all patients with a risk score of zero with excellent outcomes.

While early reports of ICI-myocarditis estimated a mortality of 50%, this study's lower event rate is consistent with contemporary reports that include cases with less severe disease.^{9,11,21,22} To date, few cohort studies have analyzed ICI-myocarditis outcomes with sample sizes ranging from 33 to 147. All studies used a major adverse cardiac event (MACE) primary outcome, typically including cardiovascular death and complete heart block, but not necessarily ventricular arrhythmias and/or cardiogenic shock.^{9,14,23-28} Over 1-5 months follow-up, 23-64% MACE was observed, similar to our discovery cohort's major cardiomyotoxic event rate of 33% and the validation cohorts' rates of 30% (SU). The study used a 30-days surveillance period which reduced the cumulative event rate but prevented misattribution of late endpoints in this multimorbid population.

With 748 patients, this is the largest cohort of ICI-myocarditis patients ever studied. Unlike previous studies, this cohort also included patients with mild or sub-clinical ICI-myocarditis (-20% of the cohort) unraveling that milder asymptomatic cases also exist.^{5,21} In contrast to previous studies which could only evaluate a limited number of predictors, the study's size allowed for a large multivariable analysis including variables implicated in previous studies as well as novel predictors. Consequently, previous associations with severe outcomes such as combination ICI therapy or corticosteroid use, were not found to be significant.^{9,24,26,27} These findings reframe combination ICI therapy as a risk factor for developing myocarditis with little prognostic value when other variables are considered.^{11,29,30} The lack of benefit with corticosteroids is provocative given their recommended use. In the discovery cohort, it is likely that immunosuppression was prescribed to sicker patients at higher risk for adverse events. On the other hand, the SU prospective validation cohort followed a pre-defined therapeutic algorithm where patients with risk score of 0 were managed without any immunosuppressants and had no major cardiomyotoxic events at one month. It is worth considering these findings in light of ongoing debates over the appropriate corticosteroid dose in ICI-myocarditis versus other irAE, high rates of corticosteroid resistance, and recent evidence of very high mortality in severe cases when corticosteroids are used at high doses as first line therapy.^{5,31,32} Troponin release is inherent to the inflammatory myocyte necrosis of myocarditis and has been demonstrated as a surrogate for disease severity in smaller studies and in multiple other cardiac conditions.^{9,18,24} However, this study demonstrate for the first time that even with a level of troponin up to 20-fold the ULN, patients with no other signs of severity (based on the risk score) have no risk of MACE. While univariate analysis of creatine kinase showed significant association in this and other studies, the multivariate model did not, suggesting it may be confounded by other biomarkers, namely troponin.^{18,33} While only 14/748 (2%) of patients in this study received ICI for active thymoma, these patients had nearly 4-fold higher severe cardio-myotoxicity risk after multivariable adjustment. This is consistent with increased autoimmunity seen in thymic neoplasms and make us wonder if ICI-myocarditis is associated with thymic dysregulation and generation of auto-reactive T-cells targeting cardio-muscular antigens and unleashed by ICI. Similarly, in retrospect, the prognostic value of low-voltage can be explained as immune infiltrates have been shown to potentially impact myocyte electrophysiology and induce arrhythmias.^{14,37}

The results of this analysis are compelling given their clinical utility. Factors such as troponin, symptoms, ECG features, LVEF and thymoma status (and likely associated dys-immunity, evaluated by production of anti-AchR and/or anti-antitin auto-antibodies) can be promptly ascertained in ambulatory and emergency room settings. While advanced imaging or endomyocardial biopsy results may be linked to outcomes, the inaccessibility of these resource-intensive diagnostics makes them impractical potential prognostic tools.^{23,25,38} Separately, this study's identification of risk profiles complements ongoing efforts to optimize immunosuppression for the treatment of ICI-myocarditis. For example, while ruxolitinib and abatacept have been effectively used to treat severe ICI-myocarditis, effects of withholding immunosuppression in asymptomatic cases is unkown. Two prospective randomized studies are currently testing abatacept treatment in ICI-myocarditis. While one trial only includes patients with suspected mild to moderate ICI-myocarditis without ventricular arrhythmias, high-degree atrio-ventricular blocks, or cardiogenic shock (NCT05335928), the other aims to optimize abatacept dosing in definite ICI-myocarditis with these latter severe features (NCT05195645). Using predictive risk scores like the ones herein presented in future studies may identify patients with the most potential for positive treatment effect, and deserve validation.

There are multiple potential clinical applications of this risk score. A score of 0 in combination with thorough clinical assessment may identify patients who can be managed with minimal or no immunosuppression since cardio-myotoxic events are rare in this group. Since over 14% of patients with a risk score of 1 had major cardio-myotoxic events in the studied cohorts, inpatient monitoring and starting milder immunosuppressant regimen (i.e monotherapies of ruxolitinib, or abatacept, or corticosteroids) may be preferred for a score of 1. Patients with score equal or above 2 may benefit most from tight in-patient monitoring and intense empiric immunosuppression (dual or triple immunossupressant therapy) due to their >30% event rate. Further studies of this risk score may allow much-needed decision support for clinicians managing this rare and complex disease entity.

In conclusion, a large multicenter registry was used to study predictors of severe outcomes in ICI-myocarditis and establish a risk score. This risk score was validated in an independent cohort, and used to guide immunosuppressive treatment. This contemporary cohort continues to show a high frequency of severe cardio-myotoxicity, occurring in one third of patients over the first month. A multivariable model showed that magnitude of troponin increase, pre-existing thymoma, presence of symptoms, low LVEF, and low QRS-voltage were associated with severe cardio-myotoxicity. These findings were used to derive a practical risk score which performed well in predicting adverse events in patients who present with ICI-myocarditis.

### Example 5. Treatment of patients

The method as disclosed in examples 1 to 3 has been performed on 30 patients with ICI-induced cardiomyotoxicity.

Patients with an end-result (score) equal to 0 did not receive any immunosuppressive treatment based on corticosteroids alone, or combined with abatacept and/or ruxolitinib.

Patients with an end-result (score) equal to 1 have been treated by corticosteroids alone, combined or not with abatacept and ruxolitinib, according to the regimen disclosed in the specification.

Patients with an end-result (score) equal or higher than 2 have been treated by a combination of abatacept, ruxolitinib and oral corticosteroid, according to the regimen disclosed in the specification.

The expected percentage of MACE in this population (patients with ICI-induced cardiomyotoxicity) was about 30% within the first 30 days after admission to hospital.

After treatment, no MACE was observed for patients with a score equal to 0 (n=7) without any immunosuppressive treatment.

All other patients recovered from their ICI-induced myotoxicity on at least one immunosuppressive treatment, with resolution of MACE if present before initiation of the treatment, and no appearance of new MACE.

The expected mortality at 30-days for these patients with ICI-induced cardiomyotoxicity was 25-60%. To date, no patient died at 30-days after being treated for patients with score ≥1 or without treatment for patient with score = 0.

### REFERENCES

1. Haslam A, Prasad V. JAMA network open. 2019;2(5):e192535-e192535.
2. Champiat et al. Ann Oncol. 2016;27(4):559-574.
3. Postow et al. N Engl J Med. 2018;378(2):158-168.
4. Zhang et al. JACC CardioOncol. 2021;3(1):35-47.
5. Salem et al. Cancer Discov. Published online February 23, 2023. doi:10.1158/2159-8290.CD-22-1180
6. Johnson et al. N Engl J Med. 2016;375(18):1749-1755.
7. Wang et al. JAMA Oncol. 2018;4(12):1721-1728.
8. Axelrod et al. Nature. 2022;611(7937):818-826.
9. Mahmood et al. J Am Coll Cardiol. 2018;71(16):1755-1764.
10. Lehmann et al. JAMA Cardiol. 2021;6(11):1329-1337.
11. Salem et al. Lancet Oncol. 2018;19(12):1579-1589.
12. Hu et al. Cardiovasc Res. 2019;115(5):854-868.
13. Bonaca et al. Circulation. 2019;140(2):80-91.
14. Power et al. Circulation. 2021;144(18):1521-1523.
15. Power et al. Arch Cardiovasc Dis. Published online April 27, 2022. doi:10.1016/j.acvd.2022.03.003
16. Niimura et al. J Clin Pharmacol. Published online December 1, 2022. doi:10.1002/jcph.2187
17. Saygin et al. J Clin Rheumatol. 2022;28(7):367-373.
18. Lehmann et al. Circulation. Published online June 15, 2023. doi:10.1161/CIRCULATIONAHA.123.062405
19. Itzhaki Ben Zadok et al. JACC CardioOncol. 2023;5(6):732-744.
20. Sterne et al. BMJ. 2009;338:b2393.
21. Palaskas et al. Eur J Heart Fail. 2021;23(10):1725-1735.
22. Ederhy et al. Arch Cardiovasc Dis. 2022;115(2): 114-116.
23. Cadour et al. Radiology. 2022;303(3):512-521.
24. Zlotoff et al. Journal for immunotherapy of cancer. 2021;9(3). doi:10.1136/jitc-2020-002007
25. Thavendiranathan et al. J Am Coll Cardiol. 2021;77(12):1503-1516.
26. Zhang et al. Eur Heart J. Published online February 2020. doi: 1 0.1 093/eurheartj/ehaa051
27. Zhang et al. Circulation. 2020;141(24):2031-2034. doi:10.1161/CIRCULATIONAHA.119.044703
28. Drobni et al. J Am Heart Assoc. 2020;9(23):e018306.
29. Naqash et al. J Clin Oncol. 2022;40(29):3439-3452.
30. Wei et al. Cancer Discov. Published online November 2020. doi:10.1158/2159-8290.CD-20-0856
31. Mason et al. N Engl J Med. 1995;333(5):269-275.
32. Wang et al. Front Pharmacol. 2021;12:770631.
33. Vasbinder et al. JACC CardioOncol. 2022;4(5):689-700.
34. Cho et al. J Clin Orthod. 2017;35(15_suppl):8521-8521.
35. Shelly et al. Cell Mol Immunol. 2011;8(3):199-202.
36. Fenioux et al. Nat Med. Published online October 26, 2023. doi:10.1038/s41591-023-02591-2
37. Hulsmans et al. Cell. 2017;169(3):510-522.e20.
38. Champion and Stone. Mod Pathol. 2020;33(1):99-108.

## Claims

1. A method for determining whether a patient under immune checkpoint inhibitor treatment and presenting an induced myotoxicity (including a myocarditis or a myositis potentially mimicking myasthenia gravis) has an increased risk of having a major cardio-myotoxic event, comprising
- assigning a value to clinical, biological and/or radiological markers obtained from the patient
- combining the values through a mathematical function,
- obtaining an end-value,
wherein the end-value is indicative of a risk of occurrence of a major cardio-myotoxic event.

2. The method of claim 1, wherein the clinical markers comprise presence of active thymoma in the patient, presence of cardio-muscular symptoms, voltage on presenting electrocardiogram (preferably QRS voltage), left ventricular ejection fraction and magnitude of troponin elevation.

3. The method of claim 2, wherein the value is
- + 2 in the presence of active thymoma, 0 in the absence of active thymoma
- + 1 in the presence of cardio-muscular symptoms, 0 in the absence of cardio-muscular symptoms
- +1 if left ventricular ejection fraction < 50%, 0 if left ventricular ejection fraction ≥ 50%
- + 1 if presence of low voltage (defined by a voltage value ≤ 0.5mV of the sum of the absolute values of the maximal values of R wave in V5 or V6 and of S wave in V1) on presenting electrocardiogram, 0 if absent
- 0 if cardiac troponin circulating level is ≤20-fold of the 99th percentile normal upper limit of the assay, + 1 if troponin level is > 20-fold and 200-fold (included) of the 99th percentile normal upper limit of the assay, + 2 if troponin level is > 200-fold and 2000-fold (included), + 3 if troponin level is > 2000-fold of the 99th percentile normal upper limit of the assay.

4. The method of any one of claims 1 to 3, wherein the clinical markers further comprise measure of presence of anti-AChR (acetylcholine receptor) auto-antibodies and/or presence of anti-Titin antibodies.

5. The method of any one of claims 1 to 4, wherein the mathematical function is the sum of the values.

6. Abatacept for use for the treatment of ICI-induced myotoxicity in a patient, wherein the end value obtained when applying the method of any one of claims 1 to 5 to the patient is indicative of a risk of MACE.

7. Abatacept for use for the treatment of ICI-induced myotoxicity in a patient, wherein the end value obtained when applying the method of claim 5 to the patient is higher or equal to 1.

8. Abatacept for use according to claim 6 or 7, in combination with a JAK inhibitor and/or a corticosteroid.

9. JAK inhibitor for use for the treatment of ICI-induced myotoxicity in a patient, wherein the end value obtained when applying the method of any one of claims 1 to 5 to the patient is indicative of a risk of MACE.

10. JAK inhibitor for use for the treatment of ICI-induced myotoxicity in a patient, wherein the end value obtained when applying the method of claim 5 to the patient is higher or equal to 1.

11. JAK inhibitor for use according to claim 9 or 10, in combination with abatacept and/or a corticosteroid.

12. Abatacept in combination with JAK inhibitor and possibly corticosteroid for use for the treatment of ICI-induced myotoxicity in a patient, wherein the end value obtained when applying the method of claim 5 to the patient is higher or equal to 2.

13. Abatacept in combination with JAK inhibitor and possibly corticosteroid for use according to claim 12, wherein
- treatment is initiated at Day 0 by a single administration of abatacept, and a daily administration of the JAK inhibitor and the corticosteroid
- at Day 5±2, clinical situation of the patient is evaluated and treatment comprises another single administration of abatacept, adjusting (maintaining, increasing or decreasing/weaning) JAK inhibitor and/or corticosteroid treatment according to respectively stabilization, worsening or improvement of the clinical situation of the patient,
- at Day 14±2, clinical situation of the patient is evaluated and treatment comprises another single administration of abatacept, adjusting (maintaining, increasing or decreasing/weaning) JAK inhibitor and/or corticosteroid treatment according to respectively stabilization, worsening or improvement of the clinical situation of the patient,
- from Day 21±2 to Day 120±2, performing assessment every 5 to 15 days of the clinic-biological situation of the patient and step by step lowering until complete weaning of corticosteroid and JAK inhibitor dosage in case of improvement, maintaining corticosteroid and JAK inhibitor dosage and administering abatacept in case of worsening and lowering corticosteroid dosage, maintaining JAK inhibitor dosage and administering abatacept in case of stability.

14. Abatacept in combination with JAK inhibitor and possibly corticosteroid for use according to claim 12 or 13, wherein
- Abatacept is administered at starting dosage of 5 to 25 mg/kg IV at day 1
- Prednisone is administered at a starting dosage of 0.1 to 1 mg/kg per day per os
- Ruxolitinib is administered at a starting dosage of 5 to 30 mg twice a day per os.

15. Abatacept in combination with JAK inhibitor and possibly corticosteroid for use according to any one of claims 12 to 14, which has a duration of up to 90 days.

16. Abatacept alone or in combination with JAK inhibitor and/or corticosteroid for use according to any one of claims 7, 8 or 12 to 15, wherein dosage of abatacept is adjusted to reach a saturation rate of CD86 receptors on circulating monocytes (CD86RO) of at least 80% within 72 hours of administration of each abatacept dosing.
